# EUROPEAN PATENT SPECIFICATION

(11) **EP 2 586 777 B1**
(45) Date of publication and mention of the grant of the patent: **12.11.2014**
(21) Application number: 12188922.4
(22) Date of filing: 18.10.2012
(51) Int. Cl.: C07D 401/04, C07F 3/02

(54) **Process for the preparation of 2-alkoxy-5-(pyridin-2-yl)pyridine, an intermediate of perampanel**
Verfahren zur Herstellung von 2-Alkoxy-5-(Pyridin-2-yl)-Pyridine, ein Zwischenprodukt von Perampanel
Procédé pour la préparation de 2-alkoxy-5-(pyridin-2-yle)-pyridine, an intermédiaire de Perampanel

(30) Priority: 27.10.2011 IT MI20111952
(43) Date of publication of application: 01.05.2013
(73) Proprietor: F.I.S.- Fabbrica Italiana Sintetici S.p.A., 36075 Alte di Montecchio Maggiore, (VI) (IT)
(72) Inventor: Fontana, Francesco, I-22030 Longone al Segrino, COMO (IT); Stabile, Paolo, I-37100 VERONA (IT)
(74) Representative: Long, Giorgio

(56) References cited:
- EP-A1- 1 553 086
- EP-A1- 2 123 639
- WO-A1-2011/031754
- LUTZ ACKERMANN ET AL: "Kumada Corriu Cross-Couplings with 2-Pyridyl Grignard Reagents", CHEMISTRY - A EUROPEAN JOURNAL, WILEY - V C H VERLAG GMBH & CO. KGAA, WEINHEIM, DE, vol. 16, no. 11, 15 March 2010 (2010-03-15), pages 3300-3303, XP007918277, ISSN: 0947-6539, DOI: 10.1002/CHEM.201000032 [retrieved on 2010-02-18]
- PAUL R PARRY ET AL: "Functionalized Pyridylboronic Acids and Their Suzuki Cross- Coupling Reactions to Yield Novel Heteroarylpyridines", JOURNAL OF ORGANIC CHEMISTRY, ACS, US, vol. 67, no. 21, 21 September 2002 (2002-09-21), pages 7541-7543, XP008136759, ISSN: 0022-3263, DOI: 10.1021/JO020388B
- DATABASE PubChem Compound [Online] 14 March 2007 (2007-03-14), XP002687241, retrieved from NCBI Database accession no. 15975233

## Description

### Background of the invention

The present invention relates to a process for synthesising 2-alkoxy-5-(pyridin-2-yl)pyridine which is an intermediate of the synthesis of the active substance Perampenel.

### State of the art

Perampanel is a pharmaceutical active substance, currently in clinical phase 3, used to treat Parkinson's disease, epilepsy and multiple sclerosis.

Perampanel, having the following chemical formula 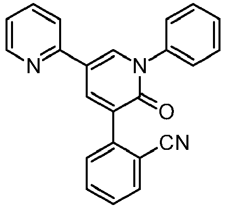 is also known as E 2007, ER 155055-90 and 3-(2-cyanophenyl)-1-phenyl-5-(2-pyridil)-1,2-dihydropyridin-2-one

Various methods of synthesis of such molecule are known, such as those reported in the patent publications EP1300396, EP1465626, EP1772450, EP1764361 and EP 1970370.

Many of the methods of synthesis of such active substance reported by the prior art use the key intermediate 5-(2-pyridil)-1,2-dihydropyridin-2-one also known as 2,3'-bipyridin-6'(1'H)-one having the following chemical formula: 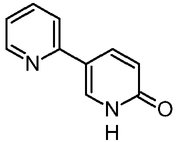 or use the synthetic precursor thereof named 2-methoxy-5-(pyridin-2-yl)pyridine or 6'-methoxy-2,3'-bipyridine having the formula: 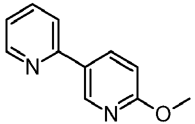 2,3'-bipyridin-6'(1'H)-one is in fact prepared by simple acid-catalysed demethylation of the 6'-methoxy-2,3'-bipyridine as thoroughly reported in the prior art.

Various ways of synthesising 2-methoxy-5-(pyridin-2-yl)pyridine are known. The process summarised in the diagram (I) below is described in the publication WO 2001096308:

### Diagram (I)

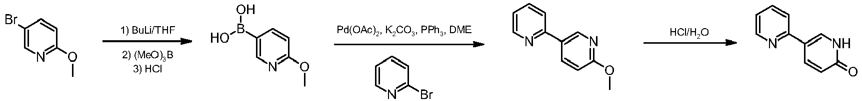

Such process highlights clear disadvantages such as the need to operate in cryogenic conditions (T=-78°C) using special equipment and the need to isolate the boronic acid via work-up; in addition the use of 2-Bromopyridine is envisaged, which is less convenient as regards the production of waste compared to 2-chloropyridine.

Another process described in WO 2004009553 is summarised in the diagram (II) :

### Diagram (II)

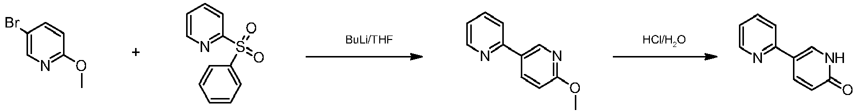

It presents clear disadvantages such as the use of high molecular weight benzenesulfonyl pyridine entailing a scarce atom-economy of the process and the need to operate at low temperature T (-78°C) using special equipment.

Lastly, a completely different process is described in WO20087093392 for the preparation of 2,3'-bipyridin-6'(1'H)-one which however does not include the preparation of the intermediate precursor named 2-methoxy-5-(pyridin-2-yl)pyridine, process shown in the diagram (III) :

### diagram (III)

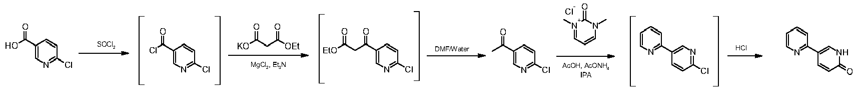

Furthermore, reference is made to Lutz Ackermann et al., "Chemistry - A European Journal", 16(11), 2010, pages 3300-3303.

### Summary of the invention

The problem addressed by the present invention is therefore to provide an alternative process for the preparation of 2-alkoxy-5-(pyridin-2-yl)pyridine having the formula (I) or a salt thereof: 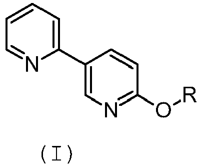 wherein R is a linear or branched C1-C6 alkyl group making it possible to overcome at least partially the drawbacks mentioned above with reference to the prior art.

Such problem is resolved by a process for the synthesis of 2-alkoxy-5-(pyridin-2-yl)pyridine of formula (I) or of a salt thereof as delineated in the attached claims, the definition of which form an integral part of this description.

Further characteristics and advantages of the process according to the invention will be evident from the description below of preferred embodiments, made by way of a non-limiting example.

### Detailed description of the invention

The present invention relates to a process for the preparation of 2-alkoxy-5-(pyridin-2-yl)pyridine of formula (I) or of a salt thereof: 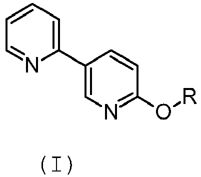 wherein R is a linear or branched C1-C6 alkyl group, comprising the following steps:
(a) conversion of a 5-halogen-2-alkoxypyridine of formula (IV) 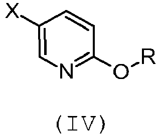 in which X is chosen between chlorine, bromine and iodine and R is a linear or ramified C1-C6 alkyl group,
   into a halide of (6-alkoxypyridin-3-yl)magnesium of formula (III) or in a lithium chloride complex thereof having the formula (III-bis) : 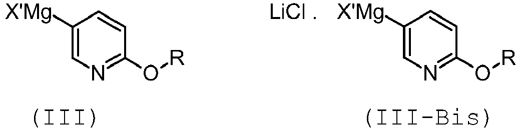 in which X' is chosen between chlorine, bromine and iodine, in which step (a) is carried out by means of a Grignard reagent or a Lithium chloride complex thereof or by means of metallic magnesium,
(b) reaction of the compound obtained in the step (a) with a 2-halogenpyridine or 2-pseudohalogenpyridine of formula (II) 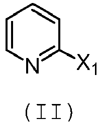 in which X1 is chosen between chlorine, bromine, iodine, triflate, nonaflate, mesylate, tosylate and besylate or -O(C=O)NR'₂ wherein R' is a linear or branched C₁-C₄ alkyl group or is phenyl or benzyl, in which step (b) is catalyzed by a metallic complex of palladium, nickel, iron or cobalt, to provide the compound of formula (I) or a salt thereof.

The group R is a linear or branched C1-C6 alkyl group, i.e. it is chosen from methyl, ethyl, n-propyl, isopropyl, n-butyl, sec-butyl, tert-butyl, n-pentyl, sec-pentyl, isopentyl, neopentyl, n-hexyl, sec-hexyl etc.

In a preferred embodiment, the process according to the present invention uses 2- Chloropyridin, i.e. the compound having the formula (II) wherein X1 is chlorine.

In a more preferred embodiment, the process according to the present invention uses 2-chloropyridin, i.e. the compound having the formula (II) wherein X1 is chlorine, and the 5-bromo-2-alkoxypyridine, i.e. the compound having the formula (IV) wherein X is bromide.

When the substituent X1 of the compound having the formula (II) is chosen from triflate, nonaflate, mesylate, tosylate and besylate, the bond between such groups and the pyridine ring is performed by means of an atom of oxygen belonging to the sulphonate group.

When X1 is -O(C=O)NR'₂, R' is a linear or branched C₁-C₄ alkyl substituent, i.e. it is chosen from methyl, ethyl, n-propyl, isopropyl, n-butyl, sec-butyl, tert-butyl, or is phenyl or benzyl.

The process according to the present invention and in particular step (b) is carried out by means of the catalysis performed by a metallic complex of palladium, nickel, iron or cobalt.

According to a preferred embodiment, step (b) is performed using as a catalyst the palladium complex having the formula Pd(dppf)Cl₂ or solvates thereof, i.e. Palladium bis-diphenylphosphinoferrocene dichloride or solvates thereof. A typical solvate of this catalyst is that formed with dichloromethane.

The metallic complex used in step (b) is used in a molar amount of about 0.1% to 5% , preferably in molar amount of about 0.1% to 1% with respect to the compound of formula (II). Even more preferably about 0.25% mol of catalyst is used with respect to the compound of formula (II).

The process according to the present invention provides that step (b) is conducted in an organic solvent chosen from tetrahydrofuran, methyl-tetrahydrofuran, toluene and mixtures thereof.

Moreover, step (b) is carried out in a temperature range of 25 to 75°C and preferably is carried out between 45 and 50°C. In the first temperature range, step (b) is completed in a time ranging from 1 to 5 hours, while operating at 45-50°C the reaction is completed in 2-4 hours.

Step (b) is carried out using from 1.1 to 1.7 molar equivalent of Grignard reagent having the formula (III) or (III-bis) with respect to the 2-halogen pyridine or pseudo halogen pyridine having the formula (II) preferably 1.1 to 1.6 molar equivalent of Grignard reagent is used and even more preferably 1.2 to 1.5 molar equivalent is used.

The process according to the present invention provides in step (a) the formation of the Grignard reagent having the formula (III) or (III-bis) by means of the reaction between 5-halogen-2-alkoxypyridine having the formula (IV) and a Grignard reagent or a lithium chloride complex thereof or by means of metallic magnesium.

To such purpose, Grignard reagents such as isopropyl magnesium chloride, t-butyl magnesium chloride and relative lithium chloride complexes thereof may be conveniently used as Grignard reagents for the halogenmetal exchange. Isopropyl magnesium chloride and the relative lithium chloride complex thereof are particularly preferred.

The Grignard reagent complexes with lithium chloride are particularly preferred in that they enable the preparation of intermediates having the formula (III-bis) with greater conversions (99% against 94%), using less reagent and in shorter times than the corresponding Grignard reagents without complexes.

If the 5-halogen-2-alkoxypyridine having the formula (IV) has a halogen group X other than the halogen of the Grignard reagent or its lithium chloride complex used for the reaction, the corresponding product having the formula (III) or (III-bis) will have a group X' which could be the same as the starting halogen-2-alkoxypyrdine or alternatively X' could be the halogen of the Grignard reagent used or alternatively a mixture of the compounds with the two different halogens could be obtained. In all cases the reaction products are understood as part of the present invention.

The synthesis of Grignard reagents by means of halogenmetal exchange, i.e. by using another Grignard reagent, according to the common general knowledge in the organic chemistry field, is typically conducted at low temperatures, i.e. at temperatures below 0°C up to 20°C. However, in the present case, it has been unexpectedly found that in the step (a) different and more severe conditions are required to allow the formation of the Grignard reagent having the formula (III) or (III-bis) and in particular the step (a) needs to be conducted between 25°C and 70°C. According to a preferred embodiment step (a) is carried out at a temperature in the range from 25 to 30°C.

To prepare the Grignard reagent having the formula (III) or (III-bis) from 1.3 to 2.5 and preferably from 1.6 to 2.2 molar equivalents of a Grignard reagent or from 1.1 to 1.5 molar equivalents of a Grignard lithium chloride complex reagent, in both cases with respect to the compound of formula (IV), are respectively required.

The reaction of step (a) is completed in an interval of time from 4 to 72 hours. In particular it is completed in 4 to 15 hours when a Grignard lithium chloride complex reagent is used, while it is completed in 15 to 72 hours when a normal Grignard reagent is used.

In particular, when performing step (a) at a temperature of 25 to 30°C, in the case a Grignard lithium chloride complex reagent in amounts from 1.1 to 1.5 molar equivalent, with respect to the compound having the formula (IV), is used, the reaction is completed in at least 4-8 hours. If 1.3 to 1.35 molar equivalent of reagent is used, the reaction is completed in 5 hours. The conversion is over 98%, typically 99%, measured by HPLC.

When performing step (a) at a temperature of 25 to 30°C, in the case a normal Grignard reagent in amounts of approximately 2 molar equivalent is used, the reaction is completed in 24 hours. The conversion is typically in the order of 94%.

The HPLC (A/A%) purity of the product obtained from step (a) is generally 94 % in all cases, i.e. both using the normal Grignard reagent and the lithium chloride complexes thereof.

The best conditions for carrying out step (a) thus prove to be the use of a Grignard lithium chloride complex reagent in amounts of approximately 1.3 to 1.35 molar equivalent with respect to the compound having the formula (IV), operating at a temperature from 25 to 30°C. The reaction is completed in approximately 5 hours. Alternatively, using approximately 2.0 molar equivalent of a normal Grignard reagent, calculated with respect to the compound of formula (IV), and operating at 25 to 30°C, the reaction time is approximately 24 hours.

The process according to the present invention is preferably used to prepare 2-methoxy-5(pyridine-2-yl)pyridine, i.e. for the compound having the formula (I) wherein R is methyl.

The step (a) according to the present invention therefore allows the preparation of a halide of (6-alkoxypyridin-3-yl)magnesium of formula (III) or a lithium chloride complex thereof of formula (III-bis) : 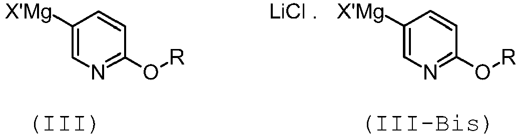 in which X' is chosen between chlorine, bromine and iodine and R is a linear or branched C1-C6 alkyl group. Therefore, the compound having the chemical name (6-methoxypyridin-3-yl)magnesium bromide constitutes an intermediate of the process according to the present invention. The compound having the chemical name (6-methoxypyridin-3-yl)magnesium chloride is also an intermediate of the present process.

The compounds chosen from the group:
a. (6- (C2-C6-alkoxy)pyridin-3-yl)magnesium chloride,
b. (6-(C2-C6-alkoxy) pyridin-3-yl)magnesium bromide,
c. (6-(C1-C6-alkoxy) pyridin-3-yl)magnesium iodide,
d. (6- (C1-C6-alkoxy)pyridin-3-yl)magnesium chloride lithium chloride complex,
e. (6-(C1-C6-alkoxy)pyridin-3-yl)magnesium bromide lithium chloride complex,
f. (6-(C1-C6-alkoxy)pyridin-3-yl)magnesium iodide lithium chloride complex;
also constitute intermediates of the process according to the present invention.

As already explained, the Grignard lithium chloride complex compounds chosen from the group:
a. (6-(C1-C6-alkoxy)pyridin-3-yl)magnesium chloride lithium chloride complex,
b. (6-(C1-C6-alkoxy)pyridin-3-yl)magnesium bromide lithium chloride complex,
c. (6-(C1-C6-alkoxy)pyridin-3-yl)magnesium iodide lithium chloride complex;
are particularly preferred.

Among the compounds belonging to the two groups listed above, the compound in which the C1-C6-alkoxy group is a methoxy group is preferred.

Such synthetic intermediates, prepared by conversion of a 5-halogen-2-(C1-C6-alkoxy)pyridine of formula (IV), which have formula (III) or formula (III-bis), are therefore useful for the preparation of 2-alkoxy-5-(pyridin-2-yl)pyridine of formula (I) in which R is a linear or branched C1-C6 alkyl group or for the preparation of a salt thereof.

The conversion of the step (b) is typically over 99% and the HPLC (A/A%) purity of the product obtained at the end of the reaction is generally around 94%.

The process according to the present invention, while considering the yields of step (a) and of step (b), enables the preparation of the compound of formula (I), at the end of the work-up, with molar yields of 89 to 92%.

It does not appear that methods permitting the product (I) to be obtained in similar yields by means of such a simple and economically advantageous process are known up to date.

Another advantage of the process according to the present invention is that the formation of the Grignard reagent (step (a)), as well as the coupling step (b) between the Grignard reagent and the 2-halogen-pyridine, are carried out at at least 25°C. Being it no longer necessary or even convenient to isolate the intermediate Grignard reagent, it is therefore possible to perform the entire process one-pot, even operating at the same temperature constantly, for example, preferably between 45 and 50°C.

The process according to the present invention makes it possible to avoid the use of special cryogenic equipments and having to work at -78°C as in the processes of the prior art.

Moreover, since the leaving groups are halogen atoms, the process according to the present invention has a high yield in terms of atom-economy in that the use of "large" leaving groups such as the benzene sulphonates, used in the processes of the prior art, can be dispensed with.

The compound of formula (I) obtained by the process according to the present invention may optionally be isolated according to the known techniques of organic synthesis and /or subjected directly to acid hydrolysis so as to form the key intermediate 5-(2-pyridil)-1,2-dihydropyridin-2-one also known as 2,3'-bipyridin-6'(1'H)-one.

In particular the compound 2-alkoxy-5-(pyridin-2-yl) pyridine of formula (I) or a salt thereof: 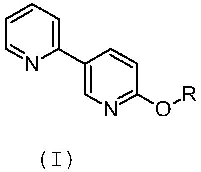 wherein R is a linear or branched C2-C6 alkyl group in which R is chosen from ethyl, n-propyl, isopropyl, n-butyl, sec-butyl, tert-butyl, n-pentyl, sec-pentyl, isopentyl, neopentyl, n-hexyl, sec-hexyl, etc. has also been shown to be useful for the preparation of 2,3'-bipyridin-6'(1'H)-one and thereby, ultimately, for the preparation of Permapanel.

To such purpose the compounds of formula (I) wherein R is an Ethyl or Isopropyl, even more preferably wherein R is Ethyl, are preferred.

The overall molar yield of the process according to the present invention carried out one-pot from the step of hydrolysis of the compound of formula (I) to give the compound 2,3'-bipyridin-6'(1'H)-one in isolated solid form is typically 81% starting from the compound of formula (II). It is estimated therefore that the step of hydrolysis of the compound of formula (I) and of isolation of the product 2,3'-bipyridin-6'(1'H)-one has a molar yield of approximately 90%. The product 2,3'-bipyridin-6'(1'H)-one thus obtained has a HPLC purity of over 99.0% (HPLC A/A%), typically 99.5%.

### EXPERIMENTAL PART

### Example 1- Synthesis of 2-methoxy-5-(pyridin-2-yl)pyridine of formula (I)

### Scheme of synthesis

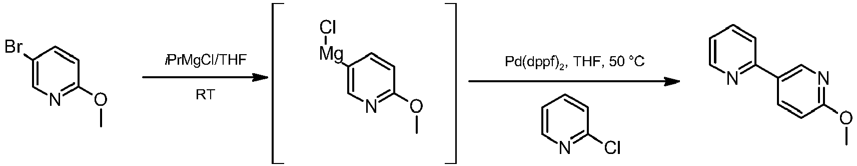

5.0 g of 5-bromo-2-methoxypyridine (1.2 equiv.), 15 ml (6 vol., with respect to 2-chloro pyridine) of anhydrous tetrahydrofuran, previously degassed for 1 hour by bubbling nitrogen through it, are put in a flask previously dried and kept under a nitrogen flow, fitted with a thermometer, a condenser and a dripper funnel. While maintaining the temperature at 25-30°C, 26.6 ml (25.3 g, 1.56 equiv.) of isopropyl magnesium chloride lithium chloride complex 1.3 M in THF are added in about 1h. The reaction medium is stirred at 25-30°C for at least 5 h. The conversion, checked via HPLC, is over 95%. 162 mg (1 mol%) of Pd(dppf)Cl₂ (1,1'-[bis(diphenylphosphino)ferrocene]dichloropalladium (II)), 2.52 g (1 equiv.) of 2-chloropyridine and 15 ml (6 vol) of previously degassed anhydrous tetrahydrofuran are loaded in another flask previously dried and kept under a nitrogen flow. The reaction medium is heated to 45-50°C and stirred for 15 min. The suspension of Grignard reagent previously prepared is transferred via a cannula into a suitable dripper funnel. The Grignard reagent is dosed in about 1 h into the flask containing 2-chloropyridine and the catalyser keeping the temperature at 45-50°C. The reaction medium is stirred at 45-50°C for at least 1 h. The conversion is checked via HPLC. 30 ml (12 vol.) of deionised water are carefully added over a period of 30 min, keeping the temperature at 20-25°C. It is stirred for 15 min., decanted and the phases are separated. The aqueous phase is extracted with two portions of 10 ml (4 vol.) of tert-butyl-methyl ether. The organic phases are combined, dried on anhydrous sodium sulphate and evaporated under a vacuum at T < 40°C, to obtain 4.6 g of wet product as a yellow oil having a wt/wt titre of 81.56%, LCAP 78.81%, equal to a molar yield of 90.7%. The raw product is used directly in the next stage of hydrolysis according to the methods of the prior art. 1H-NMR (400 MHz, CDCl₃): δ = 8.77 (d, J = 2.4 Hz, 1H), 8.70-8.69 (m, 1H), 8.28 (dd, J = 8.6, 2.5 Hz, 1H), 7.77 (m, 1 H), 7.68 (d, J = 8.0 Hz, 1H), 7.30-7.23 (m, 1H), 6.87 (d, J = 8.7 Hz, 1H), 4. 02 (s, 3H). The spectral data are in line with those shown in literature.

### Example 2- Synthesis of 2-methoxy-5-(pyridin-2-yl)pyridine of formula (I)

### Scheme of synthesis

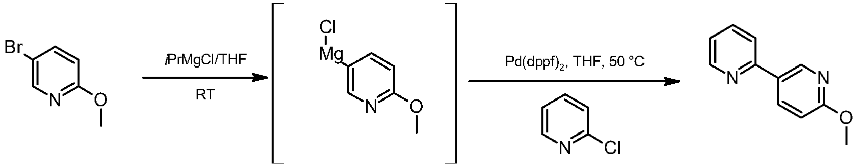

5.0 g of 5-bromo-2-methoxypyridine (1.1 equiv.), 16.5 ml (6 vol with respect to 2-chloro pyridine) of anhydrous tetrahydrofuran, previously degassed for 1 hour by bubbling nitrogen through it, are loaded in a flask previously dried and kept under a nitrogen flow, fitted with a thermometer, a condenser and a dripper funnel. While maintaining the temperature at 25-30°C, 26.6 ml (25.3 g, 1.56 equiv.) of isopropyl magnesium chloride lithium chloride complex 1.3 M in THF are added in about 1h. It is stirred at 25-30°C for at least 5 h. The conversion, checked via HPLC, is over 95%. 177 mg (1 mol%) of Pd(dppf)Cl₂ (1,1'-[bis(diphenylphosphino)ferrocene]dichloropalladium (II)), 2.74 g (1 equiv.) of 2-chloropyridine and 16.5 ml (6 vol) of previously degassed anhydrous tetrahydrofuran are loaded in another flask previously dried and kept under a nitrogen flow. The reaction medium is heated to 45-50°C and stirred for 15 min. The suspension of Grignard reagent previously prepared is transferred via a cannula into a suitable dripper funnel. The Grignard reagent is dosed in about 1 h into the flask containing 2-chloropyridine and the catalyser, while keeping the temperature at 45-50°C. It is stirred at 45-50°C for at least 1 hr. The conversion is checked via HPLC. 33 ml (12 vol.) of deionised water are carefully added, while keeping the temperature at 20-25°C, over a period of 30 min. It is stirred for 15 min., decanted and the phases are separated. The aqueous phase is extracted with two portions of 11 ml (4 vol.) of tert-butyl-methyl ether. The organic phases are washed with 11 ml (4 vol.) of sodium chloride saturate solution. The organic phases are combined, dried on anhydrous sodium sulphate and evaporated under a vacuum at T < 40°C, to obtain 3.1 g of wet product as a yellow oil having a wt/wt titre of 82.37%, LCAP 79.35%, equal to a molar yield of 56.8%.

(In this experiment there was an accidental loss of product during the separation into phases).

The raw product is used directly in the next stage of hydrolysis according to the methods of the prior art. 1H-NMR (400 MHz, CDCl₃) : δ = 8.77 (d, J = 2.4 Hz, 1H), 8.70-8.69 (m, 1H), 8.28 (dd, J = 8.6, 2.5 Hz, 1H), 7.77 (m, 1 H), 7.68 (d, J = 8.0 Hz, 1H), 7.30-7.23 (m, 1H), 6.87 (d, J = 8.7 Hz, 1H), 4.02(s, 3H). The spectral data are in line with those shown in literature.

### Example 3- Synthesis of 2-methoxy-5-(pyridin-2-yl)pyridine of formula (I)

### Scheme of synthesis

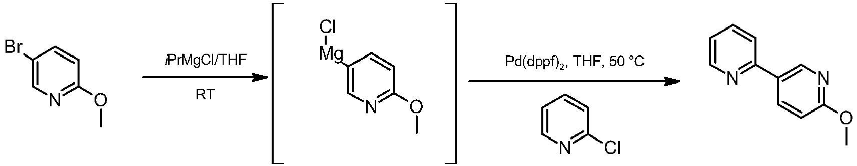

1.09 kg = 0.75 L (1.1 equiv., d = 1.453 g/ml) of 5-bromo-2-methoxy pyridine and 3.60 L (6 V) of anhydrous toluene, previously degassed under a nitrogen flow, are loaded into a reactor A previously dried under nitrogen. The mixture is heated to 25-30°C and added with 5.70 kg (1.485 equiv.) of a solution of Isopropyl magnesium chloride lithium chloride complex (20% wt/wt) in THF over about 1 h. It is stirred at 25-30°C for at least 8 hours (the reaction may be conducted overnight). The conversion is monitored via HPLC. When the reaction is complete, a mixture of 9.67 g of Pd(dppf)Cl2 (0.25 mol%), 0.60 kg equal to 0.50 1 (1 equiv., d = 1.2 g/ml) of 2-chloropyridine and 3.60 1 (6 V) of anhydrous toluene, previously degassed under a nitrogen flow, is loaded into a reactor B, previously dried and kept under a flow of nitrogen. The mixture is stirred at 45-50°C for 30 min. The suspension of Grignard reagent from reactor A is transferred into reactor B over approximately 1 hr, while keeping the T at 45-50°C. Upon completion, stirring is prosecuted for at least 4 hr at 45-50°C. The conversion is monitored in HPLC (> 99%; HPLC purity A/A% of the product >94%). When the reaction is complete, the mixture is cooled to 25-30°C, added with 3.60 L (6 V) of purified water carefully, keeping the temperature at 20-25°C, over about 30 min.-1 hr. Stirring is prosecuted for 15 min. The biphasic mixture is filtered on paper and the solid residue is washed with 0.60 L (1 V) of toluene. The filtrate is loaded into the reactor C. The organic phase (containing the reaction product) is separated. 3.60 L (6 V) of purified water are added to the organic phase. Stirring is continued for 15 min, the mixture is left to decant and the phases are separated. The organic phase (containing the reaction product) is recovered. A solution of Hydrochloric acid 4 M, prepared by using 1.41 L (2.35 V) of concentrated hydrochloric acid and 2.06 L (3.44V) of purified water, is added to the organic phase. Stirring is continued for 15 min, the mixture is left to decant and the aqueous acid phase (the product is in aqueous phase) is separated. The acid aqueous phase (containing the product) is washed twice with 1.20 L (2 x 2V) of MTBE. The aqueous phase is separated and weighed. Determination of the yield in solution of 2-methoxy-5-(pyridine-2-yl)pyridine is made by external standard HPLC analysis (molar yield in solution: 90.3%) :

### Example 4- Synthesis of 5-(2-pyridil)- 1,2-dihydropyridin-2-one or 2,3'-bipyridin-6' (1'H)-one

The acid aqueous phase containing 2-methoxy-5-(pyridin-2-yl)pyridine obtained according to Example 3 is reintroduced into the reactor C. The mixture is heated to reflux (95-100°C) for at least 4 h. The conversion is monitored in HPLC. When the reaction is complete, it is cooled to 20-25°C, the acid aqueous phase is washed with 3.54 L (5.9 V) of MTBE (the product is in aqueous phase). Stirring is continued for 15 min, the mixture is left to decant and the phases are separated. Once the phases are separated, the pH of the aqueous phase (containing the product) is adjusted to 12.5-13 with approximately 1.5 L of sodium hydroxide 30% solution. The basic aqueous phase so obtained is washed with 3.54 L (5.9 V) of MTBE (the product is in aqueous phase). Stirring is continued for 15 min, the mixture is left to decant and the phases are separated. The pH of the aqueous phase is adjusted to about 7-7.5 with approximately 0.3 L of concentrated hydrochloric acid. 7.26 L (12.1 V) of n-butanol and a solution of sodium chloride at 20%, obtained by mixing 0.67 kg (1 . 12 W) of sodium chloride and 2.68 1 (4.47 V) of purified water, are added to the aqueous phase. Stirring is continued for 15 min, the mixture is left to decant and the phases are separated (the product passes into the organic phase). The aqueous phase is again extracted with 7.26 L (12.1 V) of n-butanol. Stirring is continued for 15 min, the mixture is left to decant and the phases are separated. The organic phase so obtained are evaporated at 45-60°C under vacuum to a residue. The distillation residue is recovered with 2.4 L (4 V) of ethyl acetate. The mixture is heated to reflux and stirred for 15 min. It is cooled to 20-25 °C and stirred 30 min and then it is cooled to -10/-5°C and stirred for at least 2 h. The mixture is filtered and washed with 2x0.60 L (2x1 V) of cold ethyl acetate (-10/-5 °C). The product is dried at 45-50 °C under vacuum for at least 6 h. 736 g of product are obtained, equal to a molar yield of 80.9% from starting 2-chloro-pyridine (HPLC purity A/A% 99.40).

### Example 5- Synthesis of 2-Ethoxy-5-(pyridin-2-yl)pyridine of formula (I with R =Et)

Example 3 is repeated, but 5-bromo-2-Ethoxy pyridine (Aldrich) is used in place of 5-bromo-2-methoxy pyridine and it is performed with 5 grams of the starting compound.

2-Ethoxy-5-(pyridin-2-yl)pyridine so obtained has the following spectrum 1H-NMR (400 MHz, CDCl₃) : δ = 8.76 (dd, J = 2.5, 0.6 Hz,1H), 8 . 71-8 . 69 (m, 1H), 8.28 (dd, J = 8.6, 2.5 Hz, 1H), 7.80-7.75 (m, 1H), 7.68 (dt, J = 8.0, 1.0 Hz, 1H), 7.27-7.24 (m, 1H), 6.86 (dd, J = 8.7, 0.7 Hz, 1H), 4.46(q, J = 7.1 Hz, 2H), 1.37 (t, J = 7.1 Hz, 3H).

Subjecting 2-Ethoxy-5-(pyridin-2-yl) pyridine to the same conditions of hydrolysis as in example 4 so as to obtain 2,3'-bipyridin-6'(1'H)-one, the hydrolysis reaction is already complete in 4 hours at reflux. Such intermediate is thereby very useful for preparing 2, 3'-bipyridin-6' (1'H)-one and therefore Perampanel.

### Example 6- Synthesis of 2-Isopropoxy-5-(pyridin-2-yl)pyridine having the formula (I with R =iPr).

Example 3 is repeated, but 5-bromo-2-isopropoxy pyridine (Aldrich) is used in place of 5-bromo-2-methoxy pyridine and it is performed with 1 gram of the starting compound.

2-isopropoxy-5-(pyridin-2-yl)pyridine so obtained has the following spectrum 1H-NMR (400 MHz, CDCl₃) : δ = 8.75 (d, J = 2.3 Hz, 1H), 8.69-8.68 (m, 1H), 8.25-8.22 (m, 1H), 7.79-7.74 (m, 1H), 7.68 (dt, J = 8.0, 0.8 Hz, 1H), 7.26-7.22 (m, 1H), 6.80 (dd, J = 8.7, 0.5 Hz, 1H), 5.33 (sept, J = 6.2 Hz, 1H), 1.40 (d, J = 6.2 Hz, 6H). Subjecting 2-Isopropoxy-5-(pyridin-2-yl) pyridine to the same conditions of hydrolysis as in example 4 so as to obtain 2,3'-bipyridin-6'(1'H)-one, the hydrolysis reaction is already complete in 4 hours at reflux. Such intermediate is thereby very useful for preparing 2, 3'-bipyridin-6' (1'H)-one and therefore Perampanel.

In particular it may be appreciated how the use of the conditions of the present invention make it possible to obtain 2-alkoxy-5-(pyridine-2-yl)pyridine of formula (I) or a salt thereof, with good yields, excellent atom-economy of the process, in a single step and avoiding the use of cryogenic equipment and conditions.

## Claims

1. A process for the preparation of 2-alkoxy-5-(pyridin-2-yl)pyridine of formula (I) or of a salt thereof: 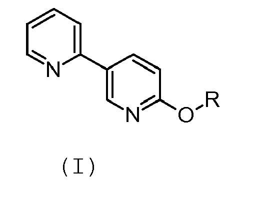 wherein R is a linear or branched C1-C6 alkyl group, comprising the following steps:
(a) conversion of a 5-halogen-2-alkoxypyridine of formula (IV) 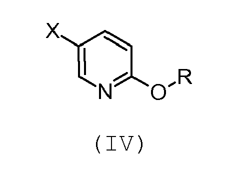 in which X is chosen between chlorine, bromine and iodine and R is a linear or branched C1-C6 alkyl group,
into a halide of (6-alkoxypyridin-3-yl)magnesium of formula (III) or in a lithium chloride complex thereof of formula (III-bis) : 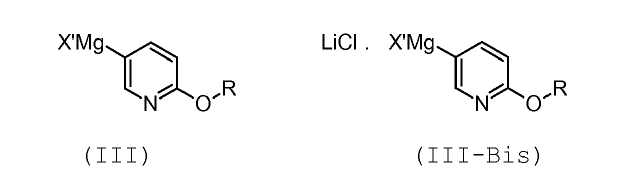 in which X' is chosen between chlorine, bromine and iodine, in which step (a) is carried out by means of a Grignard reagent or a Lithium chloride complex thereof or by means of metallic magnesium,
(b) reaction of the compound obtained in the step (a) with a 2-halogenpyridine or 2-pseudohalogenpyridine of formula (II) 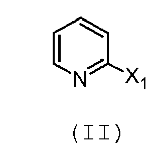 in which X1 is chosen between chlorine, bromine, iodine, triflate, nonaflate, mesylate, tosylate and besylate or -O(C=O)NR'₂ wherein R' is a linear or branched C₁-C₄ alkyl group or is phenyl or benzyl, in which step (b) is catalyzed by a metallic complex of palladium, nickel, iron or cobalt,
to provide the compound of formula (I) or a salt thereof.

2. The process according to claim 1 in which X1 is chlorine.

3. The process according to claim 1 in which the complex of palladium is Pd(dppf)Cl₂ or solvates thereof.

4. The process according to any one of claims from 1 to 3 in which the metallic complex is used in a molar amount of about 0.1% to 1% with respect to the compound of formula (II).

5. The process according to any one of claims from 1 to 4, in which step (b) is carried out in a temperature range from 25 to 75°C.

6. The process according to claim 5, in which step (b) is carried out between 45 and 50°C.

7. The process according to claim 1, in which the Grignard reagent is chosen between Isopropyl magnesium chloride, t-butyl magnesium chloride and their lithium chloride complexes.

8. The process according to any one of claims from 1 to 7 in which step (a) is carried out between 25°C and 70°C.

9. The process according to claim 8, in which step (a) is carried out between 25 and 30°C.

10. The process according to any one of claims from 1 to 9, in which step (a) is carried out by means of 1.3 to 2.5 molar equivalents of a Grignard reagent or by 1.1 to 1.5 molar equivalents of a Grignard reagent lithium chloride complex, in both the cases with respect to the compound of formula (IV).

11. The process according to any one of claims from 1 to 10 wherein R is methyl.

12. A halide of (6-alkoxypyridin-3-yl) magnesium of formula (III) or a lithium chloride complex thereof of formula (III-bis) : 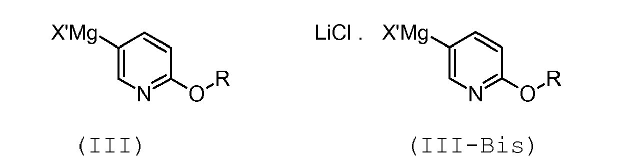 in which X' is chosen between chlorine, bromine and iodine and R is a linear or branched C1-C6 alkyl group, the said compound of formula (III) or (III-bis) being chosen from the group of:
a. (6-(C2-C6-alkoxy) pyridin-3-yl)magnesium chloride,
b. (6-(C2-C6-alkoxy) pyridin-3-yl)magnesium bromide,
c. (6-(C1-C6-alkoxy) pyridin-3-yl)magnesium iodide,
d. (6- (C1-C6-alkoxy)pyridin-3-yl)magnesium chloride lithium chloride complex,
e. ( 6-(C1-C6-alkoxy)pyridin-3-yl)magnesium bromide lithium chloride complex,
f. (6-(C1-C6-alkoxy)pyridin-3-yl)magnesium iodide lithium chloride complex.

13. The compound according to claim 12 chosen from:
a . (6-(C1-C6-alkoxy)pyridin-3-yl)magnesium chloride lithium chloride complex,
b . ( 6-(C1-C6-alkoxy)pyridin-3-yl)magnesium bromide lithium chloride complex,
c. (6-(C1-C6-alkoxy)pyridin-3-yl)magnesium iodide lithium chloride complex.

14. The compound according to claim 13 wherein the C1-C6 alkoxy group is a methoxy group.

15. A process for the preparation of a halide of (6-alkoxypyridin-3-yl)magnesium of formula (III) or a lithium chloride complex thereof of formula (III-bis) : 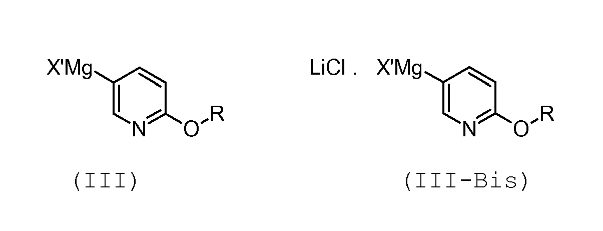 in which X' is chosen between chlorine, bromine and iodine and R is a linear or branched C1-C6 alkyl group by means of the conversion of a 5-halogen-2-alkoxypyridine of formula (IV) : 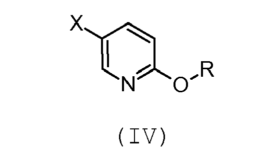 in which X is chosen between chlorine, bromine and iodine and R is a linear or branched C1-C6 alkyl group by means of a Grignard reagent or a lithium chloride complex thereof or by means of metallic magnesium.

16. The use of a halide of (6-(C1-C6-alkoxy)pyridin-3-yl)magnesium of formula (III) or of a lithium chloride complex thereof of formula (III-bis) according to any one of claims from 12 to 15 for the preparation of 2-alkoxy-5-(pyridin-2-yl)pyridine of formula (I) in which R is a linear or branched C1-C6 alkyl group or a salt thereof.

17. 2-alkoxy-5-(pyridin-2-yl)pyridine of formula (I) or a salt thereof: 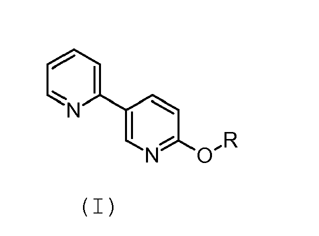 wherein R is chosen from the group consisting of ethyl, n-propyl, isopropyl, n-pentyl, sec-pentyl, isopentyl and neopentyl.

18. The use of the compound according to claim 17 for the preparation of 2,3' bipyridin-6'(1'H)-one or of Perampanel.

## Patentansprüche

1. Verfahren zur Herstellung von 2-Alkoxy-5-(pyridin-2-yl)-pyridin der Formel (I) oder eines Salzes davon: 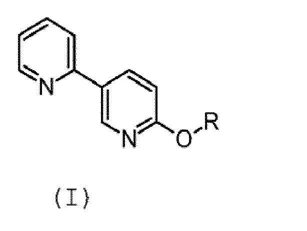 wobei R eine lineare oder verzweigte C1-C6-Alkylgruppe ist, umfassend die folgenden Schritte:
(a) Umsetzung eines 5-Halogen-2-alkoxypyridins der Formel (IV) 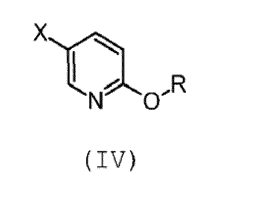 wobei X aus Chlor, Brom und lod ausgewählt ist und R eine lineare oder verzweigte C1-C6-Alkylgruppe ist,
in ein Halogenid von (6-Alkoxypyridin-3-yl)-magnesium der Formel (III) oder in einen Lithiumchlorid-Komplex davon der Formel (III-bis): 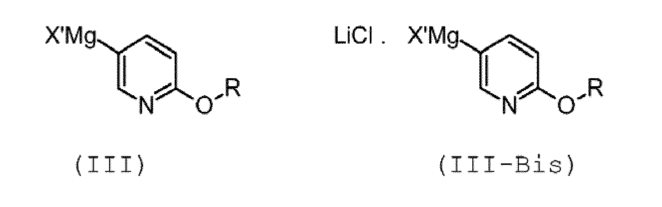 wobei X' aus Chlor, Brom und lod ausgewählt ist, wobei Schritt (a) mit Hilfe eines Grignard-Reagenz oder eines Lithiumchlorid-Komplexes davon oder mit Hilfe von metallischem Magnesium durchgeführt wird,
(b) Reaktion der in Schritt (a) erhaltenen Verbindung mit einem 2-Halogenpyridin oder 2-Pseudohalogenpyridin der Formel (II) 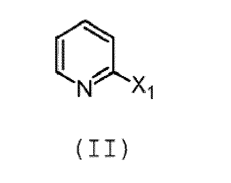 wobei X1 aus Chlor, Brom, lod, Triflat, Nonaflat, Mesylat, Tosylat und Besylat oder -O(C=O)NR'₂ ausgewählt ist, wobei R' eine lineare oder verzweigte C1-C4-Alkylgruppe ist oder Phenyl oder Benzyl ist, wobei Schritt (b) durch einen metallischen Komplex aus Palladium, Nickel, Eisen oder Kobalt katalysiert wird,
um die Verbindung der Formel (I) oder ein Salz davon bereitzustellen.

2. Verfahren nach Anspruch 1, wobei X1 Chlor ist.

3. Verfahren nach Anspruch 1, wobei der Komplex von Palladium Pd(dppf)Cl₂ oder Solvate davon ist.

4. Verfahren nach einem der Ansprüche 1 bis 3, wobei der metallische Komplex in einer molaren Menge von etwa 0,1 % bis 1% in Bezug auf die Verbindung der Formel (II) verwendet wird.

5. Verfahren nach einem der Ansprüche 1 bis 4, wobei Schritt (b) in einem Temperaturbereich von 25 bis 75°C durchgeführt wird.

6. Verfahren nach Anspruch 5, wobei Schritt (b) zwischen 45 und 50°C durchgeführt wird.

7. Verfahren nach Anspruch 1, bei dem das Grignard-Reagenz aus Isopropyl-magnesiumchlorid, t-Butyl-magnesiumchlorid und deren Lithiumchlorid-Komplexen ausgewählt ist.

8. Verfahren nach einem der Ansprüche 1 bis 7, wobei Schritt (a) zwischen 25 und 70°C durchgeführt wird.

9. Verfahren nach Anspruch 8, wobei Schritt (a) zwischen 25 und 30°C durchgeführt wird.

10. Verfahren nach einem der Ansprüche 1 bis 9, wobei Schritt (a) mit Hilfe von 1,3 bis 2,5 molaren Äquivalenten eines Grignard-Reagenz oder mit 1,1 bis 1,5 molaren Äquivalenten eines Lithiumchlorid-Komplexes des Grignard-Reagenz durchgeführt wird, in beiden Fällen in Bezug auf die Verbindung der Formel (IV).

11. Verfahren nach einem der Ansprüche 1 bis 10, wobei R Methyl ist.

12. Halogenid von (6-Alkoxypyridin-3-yl)-magnesium der Formel (III) oder ein Lithiumchlorid-Komplex davon der Formel (III-bis): 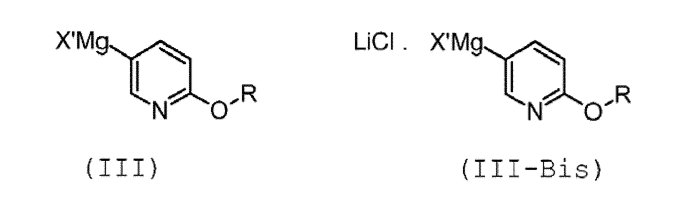 wobei X' aus Chlor, Brom und lod ausgewählt ist und R eine lineare oder verzweigte C1-C6-Alkylgruppe ist, wobei die Verbindung der Formel (III) oder (III-bis) ausgewählt ist aus der Gruppe:
a. (6-(C2-C6-Alkoxy)-pyridin-3-yl)-magnesiumchlorid,
b. (6-(C2-C6-Alkoxy)-pyridin-3-yl)-magnesiumbromid,
c. (6-(C1-C6-Alkoxy)-pyridin-3-yl)-magnesiumiodid,
d. (6-(C1-C6-Alkoxy)-pyridin-3-yl)-magnesiumchlorid-Lithiumchlorid-Komplex,
e. (6-(C1-C6-Alkoxy)-pyridin-3-yl)-magnesiumbromid-Lithiumchlorid-Komplex,
f. (6-(C1-C6-Aikoxy)-pyridin-3-yl)-magnesiumiodid-Lithiumchlorid-Komplex.

13. Verbindung nach Anspruch 12, ausgewählt aus:
a. (6-(C1-C6-Alkoxy)-pyridin-3-yl)-magnesiumchiorid-Lithiumchlorid-Komplex,
b. (6-(C1-C6-Alkoxy)-pyridin-3-yl)-magnesiumbromid-Lithiumchlorid-Komplex,
c. (6-(C1-C6-Alkoxy)-pyridin-3-yl)-magnesiumiodid-Lithiumchlorid-Komplex.

14. Verbindung nach Anspruch 13, wobei die C1-C6-Alkoxygruppe eine Methoxygruppe ist.

15. Verfahren zur Herstellung eines Halogenids von (6-Alkoxypyridin-3-yl)-magnesium der Formel (III) oder eines Lithiumchlorid-Komplexes davon der Formel (III-bis): 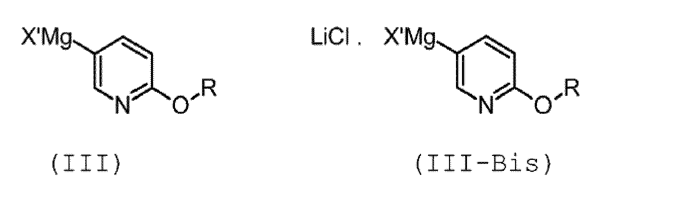 wobei X' aus Chlor, Brom und lod ausgewählt ist und R eine lineare oder verzweigte C1-C6-Alkylgruppe ist, mit Hilfe der Umsetzung eines 5-Halogen-2-alkoxypyridins der Formel (IV): 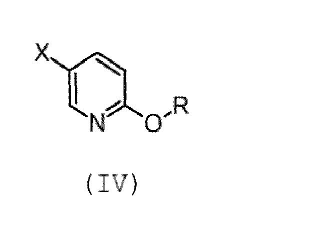 wobei X aus Chlor, Brom und lod ausgewählt ist und R eine lineare oder verzweigte C1-C6-Alkylgruppe ist, mit Hilfe eines Grignard-Reagenz oder eines Lithiumchlorid-Komplexes davon oder mit Hilfe von metallischem Magnesium.

16. Verwendung eines Halogenids von (6-(C1-C6-Alkoxy)-pyridin-3-yl)-magnesium der Formel (III) oder eines Lithiumchlorid-Komplexes davon der Formel (III-bis) nach einem der Ansprüche 12 bis 15 zur Herstellung von 2-Alkoxy-5-(pyridin-2-yl)-pyridin der Formel (I), wobei R eine lineare oder verzweigte C1-C6-Alkylgruppe oder ein Salz davon ist.

17. 2-Alkoxy-5-(pyridin-2-yl)-pyridin der Formel (I) oder ein Salz davon: 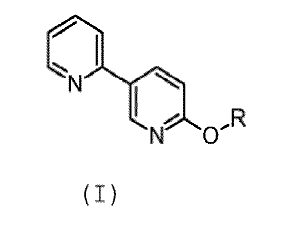 wobei R ausgewählt ist aus der Gruppe bestehend aus Ethyl, n-Propyl, Isopropyl, n-Pentyl, sec-Pentyl, Isopentyl und Neopentyl.

18. Verwendung der Verbindung nach Anspruch 17 zur Herstellung von 2,3'-Bipyridin-6'-(1'H)-on oder Perampanel.

## Revendications

1. Procédé pour la préparation de 2-alcoxy-5-(pyridin-2-yl)pyridine de formule (I) ou d'un sel de celle-ci : 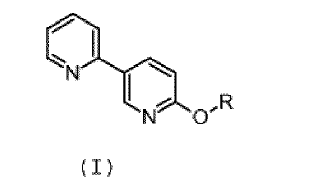 où R est un groupe alkyle en C1-C6 linéaire ou ramifié, comprenant les étapes suivantes :
(a) de conversion d'une 5-halogéno-2-alcoxypyridine de formule (IV) 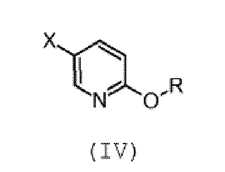 dans laquelle X est choisi parmi le chlore, le brome et l'iode et R est un groupe alkyle en C1-C6 linéaire ou ramifié,
en un halogénure de (6-alcoxypyridin-3-yl)magnésium de formule (III) ou en un complexe de chlorure de lithium de celui-ci de formule (III-bis) : 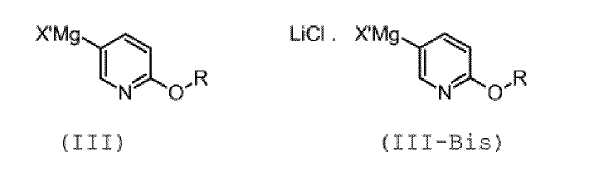 dans lesquelles X' est choisi parmi le chlore, le brome et l'iode, dans laquelle l'étape (a) est réalisée au moyen d'un réactif de Grignard ou d'un complexe de chlorure de lithium de celui-ci au moyen de magnésium métallique,
(b) de réaction du composé obtenu dans l'étape (a) avec une 2-halogénopyridine ou une 2-pseudohalogénopyridine de formule (II) 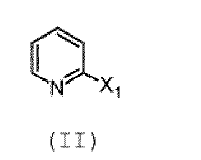 dans laquelle X1 est choisi parmi le chlore, le brome, l'iode, un triflate, un nonaflate, un mésylate, un tosylate et un bésylate ou -O(C=O)NR'₂ où R' est un groupe alkyle en C₁-C₄ linéaire ou ramifié ou est un groupe phényle ou benzyle, dans laquelle l'étape (b) est catalysée par un complexe métallique de palladium, de nickel, de fer ou de cobalt,
pour fournir le composé de formule (I) ou un sel de celui-ci.

2. Procédé selon la revendication 1, dans lequel X1 est le chlore.

3. Procédé selon la revendication 1, dans lequel le complexe de palladium est Pd(dppf)Cl₂ ou des solvates de celui-ci.

4. Procédé selon l'une quelconque des revendications 1 à 3, dans lequel le complexe métallique est utilisé dans une quantité molaire d'environ 0,1 % à 1 % par rapport au composé de formule (II).

5. Procédé selon l'une quelconque des revendications 1 à 4, dans lequel l'étape (b) est réalisée dans un intervalle de température de 25 à 75°C.

6. Procédé selon la revendication 5, dans lequel l'étape (b) est réalisée entre 45 et 50°C.

7. Procédé selon la revendication 1, dans lequel le réactif de Grignard est choisi parmi le chlorure d'isopropylmagnésium, le chlorure de t-butylmagnésium et leurs complexes de chlorure de lithium.

8. Procédé selon l'une quelconque des revendications 1 à 7, dans lequel l'étape (a) est réalisée entre 25°C et 70°C.

9. Procédé selon la revendication 8, dans lequel l'étape (a) est réalisée entre 25 et 30°C.

10. Procédé selon l'une quelconque des revendications 1 à 9, dans lequel l'étape (a) est réalisée au moyen de 1,3 à 2,5 équivalents molaires d'un réactif de Grignard ou de 1,1 à 1,5 équivalents molaires d'un complexe de chlorure de lithium de réactif de Grignard, dans les deux cas par rapport au composé de formule (IV).

11. Procédé selon l'une quelconque des revendications 1 à 10, dans lequel R est le groupe méthyle.

12. Halogénure de (6-alcoxypyridin-3-yl)magnésium de formule (III) ou d'un complexe de chlorure de lithium de celui-ci de formule (III-bis) : 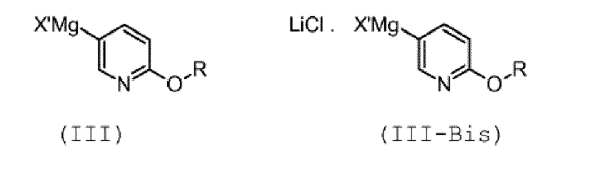 dans lesquelles X' est choisi parmi le chlore, le brome et l'iode et R est un groupe alkyle en C1-C6 linéaire ou ramifié, ledit composé de formule (III) ou (III-bis) étant choisi dans le groupe constitué de :
a. chlorure de (6-(alcoxy en C2-C6)pyridin-3-yl)magnésium,
b. bromure de (6-(alcoxy en C2-C6)pyridin-3-yl)magnésium,
c. iodure de (6-(alcoxy en C1-C6)pyridin-3-yl)magnésium,
d. un complexe de chlorure de lithium de chlorure de (6-(alcoxy en C1-C6)pyridin-3-yl)magnésium,
e. un complexe de chlorure de lithium de bromure de (6-(alcoxy en C1-C6)pyridin-3-yl)magnésium,
f. un complexe de chlorure de lithium d'iodure de (6-(alcoxy en C1-C6)pyridin-3-yl)magnésium.

13. Composé selon la revendication 12 choisi parmi :
a. un complexe de chlorure de lithium de chlorure de (6-(alcoxy en C1-C6)pyridin-3-yl)magnésium,
b. un complexe de chlorure de lithium de bromure de (6-(alcoxy en C1-C6)pyridin-3-yl)magnésium,
c. un complexe de chlorure de lithium d'iodure de (6-(alcoxy en C1-C6)pyridin-3-yl)magnésium.

14. Composé selon la revendication 13, dans lequel le groupe alcoxy en C1-C6 est un groupe méthoxy.

15. Procédé pour la préparation d'un halogénure de (6-alcoxypyridin-3-yl)magnésium de formule (III) ou d'un complexe de chlorure de lithium de celui-ci de formule (III-bis) : 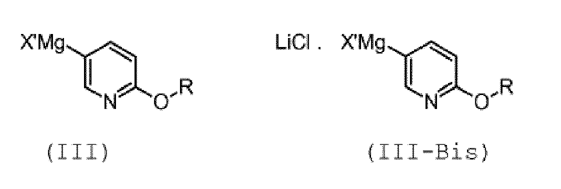 dans lesquelles X' est choisi parmi le chlore, le brome et l'iode et R est un groupe alkyle en C1-C6 linéaire ou ramifié au moyen de la conversion d'une 5-halogéno-2-alcoxypyridine de formule (IV) : 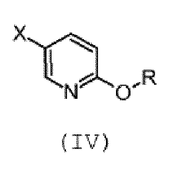 dans laquelle X est choisi parmi le chlore, le brome et l'iode et R est un groupe alkyle en C1-C6 linéaire ou ramifié au moyen d'un réactif de Grignard ou d'un complexe de chlorure de lithium de celui-ci ou au moyen de magnésium métallique.

16. Utilisation d'un halogénure de (6-(alcoxy en C1-C6)pyridin-3-yl)magnésium de formule (III) ou d'un complexe de chlorure de lithium de celui-ci de formule (III-bis) selon l'une quelconque des revendications 12 à 15 pour la préparation de 2-alcoxy-5-(pyridin-2-yl)pyridine de formule (I) dans laquelle R est un groupe alkyle en C1-C6 linéaire ou ramifié ou d'un sel de celle-ci.

17. 2-alcoxy-5-(pyridin-2-yl)pyridine de formule (I) ou sel de celle-ci : 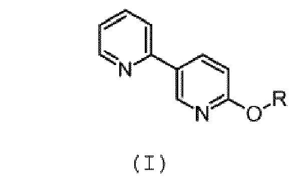 dans laquelle R est choisi dans le groupe constitué d'un groupe éthyle, n-propyle, isopropyle, n-pentyle, sec-pentyle, isopentyle et néopentyle.

18. Utilisation du composé selon la revendication 17 pour la préparation de 2,3'-bipyridin-6'(1'H)-one ou de Perampanel.
